# EUROPEAN PATENT APPLICATION

(11) **EP 4 431 601 A1**
(43) Date of publication of application: **18.09.2024**
(21) Application number: 23305370.1
(22) Date of filing: 17.03.2023
(51) Int. Cl.: C12N 9/22, C12N 15/82

(54) **METHOD FOR PRODUCING A GENE KNOCK-OUT BY TARGETED INSERTION OF STOP CODONS**

(71) Applicant: Limagrain Europe, 63360 Saint-Beauzire (FR)
(72) Inventor: BARRET, Pierre, 63360 SAINT-BEAUZIRE (FR); TASSY, Caroline, 63360 SAINT-BEAUZIRE (FR); KADNER, Maxime, 63360 SAINT-BEAUZIRE (FR)
(74) Representative: Flesselles, Bruno F.G.

(57) **Abstract**

The invention relates to methods for producing a truncated protein in a cereal, by introducing a universal stop cassette with stop codons in all six frames in the gene coding for such protein.

## Description

The invention relates to the field of molecular biology applied in plants, and the generation of plants deficient in production of a target protein. In particular, the invention relates to methods for producing a truncated protein in a cereal, by using a universal stop cassette (preferably in the form of a linear DNA segment) that is introduced in the gene coding for such protein.

Plant breeding is relevant for both crop improvement and the development of sustainable agriculture, and to address consumers concerns, which are food availability, with good prices and quality, and with a limited impact on the environment. New advances in biotechnology have the potential to accelerate plant breeding but this requires a better understanding of the functions of the genes involved in agronomically important traits. Hence, functional validation of genes is key to understand their roles in agronomical traits.

Functional validation of a candidate gene can be achieved through overexpression of the gene in a genetic background deficient for the searched character, or through knock-out (KO) of the resident candidate gene in a genetic background harboring the trait of interest. One of the most widely used methods remains the study of natural mutant following ethyl methanesulfonate (EMS) mutagenesis. EMS induces typically G>A bases changes that are randomly distributed among the genome, but no frameshifts that could be more efficient for gene KO, and moreover without any possibility to choose the position of the mutation in the gene sequence. Moreover, EMS induces thousands of off-target mutations all over the genome, from which some could potentially induce other effects and hence false positive or false negative results. One can also note the danger of using EMS (as it is a mutagen which may also have effect on the manipulator) and that the screening of EMS mutant may be difficult to conduct. Another problem, inherent to the hexaploid structure of bread wheat genome, is that it is highly difficult to identify in the same plant resulting from EMS mutagenesis three mutations having generated three knockouts in three homoeologous candidate genes for a trait. One can also induce mutagenesis with use of T-DNA mutation library, or use of RNAi constructs to silence genes of interests, but these approaches are limited by wheat transformation efficiency.

For the last ten years, genome editing using clustered regularly interspaced short palindromic repeat (CRISPR)-associated protein 9 (Cas9) genome editing system (CRISPR-Cas9) has been used to induce KO in genes.

The method was first proposed on wheat by Shan et al. (2013), and described in detail by Shan et al. (2014), and subsequently applied to a large panel of agronomic traits (for review see Zhang et al. 2021). In the CRISPR-Cas9 system, a guide sequence that matches to a specific genomic DNA is placed 5' of a synthetic RNA to constitute a single-guide RNA (sgRNA). The sgRNA/Cas complex binds to the target DNA next to the protospacer adjacent motif (PAM) via base-pairing and generates a double-strand break (DSB) in the target locus, which is repaired either by non-homologous end joining (NHEJ) or homology directed repair (HDR).

In cultured plant cells for regeneration of plantlets, NHEJ is the predominant DSB repair pathway (Li et al. 2016; Puchta 2004). This pathway is error-prone, which introduces small insertions or deletions (indels) at the break site (Gorbunova and Levy, 1997). When these indels, which are +1/-1 in 90% of the cases, are positioned in the targeted gene open reading frame (ORF), they induce a frameshift that disturbs the structure of the protein and often generate prematurely a stop codon (Itzkovitz & Alon 2007). More complex systems were developed to obtain predictable deletions (Wang et al. 2014), that induce frameshifts in the same way.

However, some problems remain unsolved. First, the frameshift generates a new, unknown succession of amino acids until occurrence of the stop codon. Depending on the position of the frameshift and of the length of the new amino acid stretch, residual or new activity of the targeted gene might be observed, which could disturb the reading of the phenotype and the assessment of the consequence of a compete loss of function of the targeted gene (Jiang et al. 2019). Secondly, for allergenicity, toxicity and regulatory concerns, the presence in the plant, and sometimes in the grain, of a new, uncharacterized protein could raise questions.

With regards to these disadvantages, and despite the high efficiency of the frameshift method performed by the CRISPR/Cas9 system, there is a need to develop other methods to obtain a precise and non-ambiguous positioning of stop codons in the ORF of candidate genes to obtain precise KO (PKO), at a target position selected beforehand.

Base editing is performed by a combination of a nickase-Cas9 (nCas9) and another associated enzyme that induces transitions of nucleotides in a short window distal from the PAM sequence (A>G (Li et al. 2018), C>T (Zong et al. 2018); for review Molla et al. 2021), The method works on wheat, but the reported efficiency of 0.3% for C>T at LOX2 locus is quite low, and implies a heavy effort to hope generate a triple mutation in the three A, B and D genomes of hexaploid wheat (Zong et al. 2017). Moreover, the window of activity, determined by the position of PAM sequence, is very short (6 bp), does not contain all the time the nucleotide to be targeted, and, even if the right nucleotide is present, transitions have low chances to generate a stop codon. For all these reasons, sites that could be targeted to generate a stop codon in an ORF are very rare (Kuscu et al. 2017), and thereby the base editing method is not completely efficient to induce precise KO in candidate genes, especially in wheat.

The prime editing (PE) method has been developed, based on the reverse transcription in the plant genome of an RNA sequence positioned at the extremity of the guide RNA (Anzalone et al. 2019; for review Molla et al. 2021). The main advantage of the PE method compared to BE is that it can perform, theoretically, all nucleotide transitions and transversions, and moreover, can induce indels. It is thus possible to consider rewriting any base, but still in a limited window around the PAM sequence (Type I window), with the need to add synonymous mutations to increase efficiency, and with a final efficiency which remains lower than that of the BE (Lin et al. 2020). Efficiency reported on wheat protoplasts for transitions or transversion in the type I window are between 0.2 and 1.5% of NGS reads presenting the mutation (Lin et al. 2020).

Just like BE, and due to the constraint of positioning close to the PAM sequence, it limits the position of the introduced stop codons. Another strategy involving PE could be the insertion of a stop codon in frame, or the replacement of a pre-existing codon by a stop codon. This latter strategy was performed on rice (Lin et al. 2020). The authors tried to replace a CCG codon by an ochre TAA stop codon, and they obtained 2.3% of rice plants with the mutation after stable transformation. This relatively low rate of editing for rice does not make it possible to consider that this strategy can successfully be performed in bread wheat, in particular if the three copies of the candidate gene must be modified.

In addition to frameshifts and the creation of stop codons by base changes or replacement, another way to perform precise KOs could be the targeted insertion of stop codons. In bread wheat, targeted deletions (Youssef et al. 2017) and insertions (Wang et al. 2014) of DNA cassettes were previously described. Moreover, on *C*. *elegans,* targeted insertion of stop codons by homologous recombination (HR) was described (Wang et al. 2018). Homologous recombination is a DNA double-strand break repair pathway which is in the minority in plants (Wang et al 2014), in which the NHEJ pathway remains predominant (Li et al. 2016; Puchta 2004). Therefore, the insertion of a cassette by NHEJ is an interesting way to explore in plants in general (for review Dong and Ronald 2020) and in wheat in particular.

The invention uses a specific multi-stop cassette which contains stop codons in all six reading frames and which can therefore be inserted within a gene of interest, and be efficient to induce a stop codon at the insertion position, whatever the frame and direction of the insertion. Preferably, such multi-stop cassette contains at most 20 nucleotides (i.e. the minimal length of the sequence is 20 nucleotides, which means that if a nucleotide of the 3' or 5' end of the sequence is deleted, the cassette will not contain stop codons in all six reading frames anymore), or preferably at most 15 nucleic acids, more preferably at most 14 nucleic acids, most preferably at most 12 nucleic acids.

A preferred multi-stop sequence is depicted as SEQ ID NO: 1 in one orientation, SEQ ID NO: 2 representing the other orientation. This sequence is 12 nucleotides long.
SEQ ID NO: 1 5' TTAATTAGTTAA 3'
SEQ ID NO: 2 5' TTAACTAATTAA 3'

Other sequences that can be used.

**Table 1. 12-nucleotides long multi-stop sequences containing stop codons in all six frames of reading**

| SEQ ID NO | Forward sequence | SEQ ID NO | Reverse sequence |
|---|---|---|---|
| 1 | 5' T**TAA**T**TAG**T**TAA** 3' | 2 | 5' T**TAA**C**TAA**T**TAA** 3' |
| 3 | 5' T**TAA**T**TAG**T**TAG** 3' | 4 | 5' C**TAA**C**TAA**T**TAA** 3' |
| 5 | 5' T**TAA**T**TAA**T**TAG** 3' | 6 | 5' C**TAA**T**TAA**T**TAA** 3' |
| 7 | 5' T**TAG**T**TAG**T**TAA** 3' | 8 | 5' T**TAA**C**TAA**C**TAA** 3' |
| 9 | 5' T**TAG**T**TAG**T**TAG** 3' | 10 | 5' C**TAA**C**TAA**C**TAA** 3' |
| 11 | 5' T**TAG**T**TAA**T**TAA** 3' | 12 | 5' T**TAA**T**TAA**C**TAA** 3' |
| 13 | 5' T**TAG**T**TAA**T**TAG** 3' | 14 | 5' C**TAA**T**TAA**C**TAA** 3' |
| 15 | 5' C**TAA**T**TAG**T**TAA** 3' | 16 | 5' T**TAA**C**TAA**T**TAG** 3' |
| 17 | 5' C**TAA**T**TAG**T**TAG** 3' | 18 | 5' C**TAA**C**TAA**T**TAG** 3' |
| 19 | 5' C**TAG**T**TAG**T**TAA** 3' | 20 | 5' T**TAA**C**TAA**C**TAG** 3' |
| 21 | 5' C**TAG**T**TAG**T**TAG** 3' | 22 | 5' C**TAA**C**TAA**C**TAG** 3' |
| 23 | 5' C**TAG**T**TAA**T**TAA** 3' | 24 | 5' T**TAA**T**TAA**C**TAG** 3' |
| 25 | 5' C**TAG**T**TAA**T**TAG** 3' | 26 | 5' C**TAA**T**TAA**C**TAG** 3' |
| 27 | 5' T**TAA**C**TAG**T**TAG** 3' | 28 | 5' C**TAA**C**TAG**T**TAA** 3' |
| 29 | 5' T**TAG**C**TAG**T**TAA** 3' | 30 | 5' T**TAA**C**TAG**C**TAA** 3' |
| 31 | 5' T**TAG**C**TAG**T**TAG** 3' | 32 | 5' C**TAA**C**TAG**C**TAA** 3' |
| 33 | 5' T**TAG**C**TAA**T**TAA** 3' | 34 | 5' T**TAA**T**TAG**C**TAA** 3' |
| 35 | 5' T**TAG**C**TAA**T**TAG** 3' | 36 | 5' C**TAA**T**TAG**C**TAA** 3' |
| 37 | 5' T**TAA**T**TAG**C**TAG** 3' | 38 | 5' C**TAG**C**TAA**T**TAA** 3' |
| 39 | 5' T**TAG**T**TAG**C**TAA** 3' | 40 | 5' T**TAG**C**TAA**C**TAA** 3' |
| 41 | 5' T**TAG**T**TAG**C**TAG** 3' | 42 | 5' C**TAG**C**TAA**C**TAA** 3' |
| 43 | 5' T**TAG**T**TAA**C**TAG** 3' | 44 | 5' C**TAG**T**TAA**C**TAA** 3' |
| 45 | 5' T**TAA**C**TAG**C**TAG** 3' | 46 | 5' C**TAG**C**TAG**T**TAA** 3' |
| 47 | 5' T**TAG**C**TAG**C**TAG** 3' | 48 | 5' C**TAG**C**TAG**C**TAA** 3' |
| 49 | 5' T**TAG**C**TAA**C**TAG** 3' | 50 | 5' C**TAG**T**TAG**C**TAA** 3' |
| 51 | 5' C**TAG**C**TAG**T**TAG** 3' | 52 | 5' C**TAA**C**TAG**C**TAG** 3' |
| 53 | 5' C**TAG**C**TAA**T**TAG** 3' | 54 | 5' C**TAA**T**TAG**C**TAG** 3' |
| 55 | 5' C**TAG**T**TAG**C**TAG** 3' | 56 | 5' C**TAG**C**TAA**C**TAG** 3' |
| 57 | 5' C**TAG**T**TAA**C**TAG** 3' | 57 | 5' C**TAG**T**TAA**C**TAG** 3' |
| 58 | 5' C**TAG**C**TAG**C**TAG** 3' | 58 | 5' C**TAG**C**TAG**C**TAG** 3' |
| 59 | 5' T**TAA**T**TAA**T**TAA** 3' | 59 | 5' T**TAA**T**TAA**T**TAA** 3' |
| 60 | 5' C**TAA**T**TAA**T**TAG** 3' | 60 | 5' C**TAA**T**TAA**T**TAG** 3' |
| 61 | 5' T**TAA**C**TAG**T**TAA** 3' | 61 | 5' T**TAA**CTAGTTAA 3' |
| 62 | 5' T**TAG**T**TAA**C**TAA** 3' | 62 | 5' T**TAG**T**TAA**C**TAA** 3' |
| 63 | 5' T**TAG**C**TAG**C**TAA** 3' | 63 | 5' T**TAG**C**TAG**C**TAA** 3' |
| 64 | 5' C**TAA**C**TAG**T**TAG** 3' | 64 | 5' C**TAA**C**TAG**T**TAG** 3' |

Other sequences can also be designed, by adequately combining ocher and amber codons in the forward and reverse sequences.

Generally, the multi-stop cassette is flanked by nucleotides, in a nucleic acid molecule that is provided to the plant cell, as described below. SEQ NO: 65 represents such a multi-stop sequence depicted as SEQ ID NO: 1 (in bold text) introduced within flanking DNA sequences. SEQ ID NO: 66 represents the complementary sequence of SEQ ID NO: 65 (and hence contains SEQ ID NO: 2 (in bold text) which is complementary to SEQ ID NO: 1).

5′ CGTGCCGCCGTTAATTAGTTAACTAAGGCACCTACG 3′ (SEQ ID NO: 65)

||||||||||||||||||||||||||||||||||||

3′ GCACGGCGGCAATTAATCAATTGATTCCGTGGATGC 5′ (SEQ ID NO: 66)

Presence of nucleotides flanking the multi-stop cassette is important, in order to take into account the fact that digestion by nucleases may occur *in vivo* when the DNA molecule containing the multi-stop cassette is introduced within the cells.

The invention thus relates to a method for modifying the genome of a plant cell, comprising
(a) providing an endonuclease, in particular a Cas nuclease directed to a target site of a gene of interest to the plant cell;
(b) providing a nucleic acid molecule comprising a multi-stop cassette that comprises stop codons in all six reading frames to the plant cell, wherein the Cas nuclease cuts the genome at the target site, and wherein fragment of the nucleic acid molecule comprising the stop codons in all six frames of reading is introduced within the plant cell's genome during repair of the genome at the cut induced by the Cas nuclease.

As shown above, the multi-stop cassette present within the nucleic acid molecule provided in (b) contains stop codons in all six frames. The nucleic acid molecule provided in (b) may contain other nucleotides extending in 5' and/or 3' (preferably in both 5' and 3') of the multi-stop cassette.

In a preferred embodiment, the plant is a cereal.

Consequently, the resulting cell comprises the multi-stop cassette comprising stop codons in all six frames of reading within its genome, which is thus modified in the target gene. Repair of the genome at the cut induced by the Cas nuclease is generally achieved by NHEJ (Non Homologous End Joining).

Since the method introduces a stop codon within the gene of interest, it thus can be used for producing a truncated protein expressed by a gene of interest. It is to be noted that the multi-stop cassette comprising stop codons in all six reading frames is introduced in the coding region of the gene of interest. It may be introduced in the first exon (to limit the size of the produced truncated protein), but is more preferably introduced downstream (such as in the second or third exon when appropriate) to avoid use of further ATG codons that could be used to reinitiate translation and to the production of a functional protein if the stop codon is introduced too close from the actual start codon of the protein. The site of integration of the sequence with the stop codon will be selected by one of skill in the art to avoid production of a truncated protein that could retain some kind of functional activity. Hence, introducing the stop codon close to the beginning of the coding sequence may lead to the production of a functional protein truncated in its N-terminus (with regards to the native protein), while production of a functional protein truncated in its C-terminus may occur if the stop codon is introduced a location that is too far from the native ATG. it is to be noted that there is some latitude in the site of integration, as the presence of the stop codons in the six frames of reading warrants that there will be a stop codon in the reading frame of the gene, and that this would lead to production of a truncated protein or polypeptide. This gives some room to select a zone that the guide RNA will recognize (unique zone of the genome, or zone that presents some similarities/identities/homologies with other zones of the genome or with zones of other genomes of the plant). It is however preferred to select a zone that is unique in the genome as it is generally not desired to have the nucleic acid molecule with the stop codons to be introduced at multiple sites of the genomes

In particular, the multi-stop cassette comprising stop codons in all six frames of reading is SEQ ID NO: 1 or SEQ ID NO: 2, or any sequence of Table 1.

In a preferred embodiment, the nucleic acid molecule is provided in a linear form. It is also envisaged to use other systems such as viroid systems, where the nucleic acid molecule would be provided in a circular form and be linearized within the cell.

In one embodiment, the nucleic acid molecule provided in (b) and containing the multi-stop cassette is a single stranded DNA molecule. In one embodiment, it is a double stranded DNA molecule. In another embodiment, it is a single stranded RNA molecule.

As indicated above, it is preferred when the size (length) of the multi-stop cassette is at most 20 nucleotides, preferably at most 16 nucleotides, more preferably at most 14 nucleotides, or at most 12 nucleotides. Table 1 provides sequences of multi-stop cassette containing 12 nucleotides (with the stop codons being presented in bold).

The nucleic acid molecule provided in (b) contains the multi-stop cassette and preferably other nucleotide in 5' and/or 3'. It is preferred when it contains at least 5 other nucleotides in both 5' and 3' of the multi-stop cassette, preferably 9 or 10 other nucleotides in 5' and in 3' of the multi-stop cassette. It is not advisable to add more than 20 nucleotides on any side of the multi-stop cassette. This makes it possible to protect the multi-stop cassette in case of *in vivo* digestions of the extremities of the nucleic acid molecule when introduced within the cell.

Consequently, when the size of the multi-stop cassette is 12 nucleotides, the size of the nucleic acid provides in (b) would range from 22 nucleotides (with 5 nucleotides added at each extremity) to about 30-32 nucleotides. It is preferred when the size of the nucleic acid molecule is thus 30 bases or more, but no more than 50-52 nucleotides.

This length is a good compromise between (1) the need to have all or part of the nucleic acid molecule provided in (b) integrated within the cell genome (being reminded that it is the actual integration of the multi-stop cassette which is desired), the efficacy of which would diminish with an increased size of the nucleic acid molecule, and (2) the fact that the extremities of the nucleic acid molecule may be digested by endonucleases within the cells before integration (notably when it is in linear form). Consequently, presence of the further nucleotides at the 5' and 3' of the multi-stop cassette allows protecting the integrity of this multi-stop cassette and ensure that it is fully integrated within the genome (where the integrated fragment is generally smaller than the provided nucleic acid molecule).

The added nucleotide may be selected to ease the design of PCR primers specific for the nucleic acid, and hence for the presence of the multi-stop cassette. In particular, since the multi-stop cassette is A/T rich, the Tm of an overlapping primer would be too low (about 40°c for a 20nt primer counting adjacent bases). The added nucleotide at the 3' and the 5' end of the multi-stop cassette are thus richer in C/G nucleotide (at least 60% more preferably at least 70% of the nucleotides added at the 3' and 5' end of the multi-stop cassette are G or C nucleotides) to design primers specific to this cassette with a Tm of about 60°c.

Since insertion of the multi-stop cassette by NHEJ is desired, the nucleotides added at its 3' and/or 5' don't need to be specifically homologous to the target site in the gene of interest, although such eventuality is not to be discarded.

The endonuclease (which is a site-directed nuclease, able to cut a DNA sequence at a desired site) provided in (a) induces a double strand break at the target site in the gene of interest. The endonuclease that is used can be selected from endonucleases known in the art. It is preferably a CRISPR Cas endonuclease, most preferably a Class 2 type II (Cas9) endonuclease. One can use a Cas9 originating from any known host, such as *Streptococcus pyogenes* (SpCas9, SEQ ID NO: 67) *Staphylococcus aureus* (SaCas9), *Campylobacter jejuni* (CjCas9), *Streptococcus thermophilus* (St1Cas9), *Neisseria meningitidis* (MnCas9), *Francisella novicida* (FnCas9), E1369R/E1449H/R1556A triple mutant of *Francisella novicida* (RHA FnCas9), improved Cas9 (xCas9 described by Hu et al, Nature. 2018 Apr 5;556(7699):57-63), or Cas9-NG (Ren et al, Molecular Plants, 12 (7), 2019, 1015-1026). The endonuclease to be used in the methods herein disclosed preferably generates blunt ends when cleaving the target sequence. However, use of an endonuclease generating cohesive ends when cleaving the target sequence is also envisaged.

The endonuclease (preferably the Cas protein) is preferably associated with Nuclear Localization Signals (NLS) like the SV40 NLS or the XINucleoplasmin NLS. The NLS can be situated at any one or at both ends of the endonuclease protein, notably at the N-terminus of the endonuclease.

The NLS ensures that the endonuclease is transported in the nucleus where insertion of the multi-stop cassette takes place.

The endonuclease and nucleic acid molecule comprising the multi-stop cassette having stop codons in all six frames of reading can be provided to the nucleotide sequence of interest by multiple ways.

It is preferred when the endonuclease reaches the target site using the CRISPR system, by ways a guide RNA (which is further described below). Introduction of the protein and guide RNA within the cells is to be obtained so they can act in the cell nucleus. It can be done directly as RiboNucleoprotein (RNP) (the protein and guide RNA are pre-assembled and directly introduced within the cells) or indirectly (vectors are introduced within the cells and the protein and guide RNA are produced inside the cells). One can also deliver the protein, nucleic acid (DNA, mRNA) coding for the protein or for the guide RNAs using conjugation Cell Penetrating Peptides (CPP), nanoparticles, or biolistics. Thus, providing the endonuclease is preferably performed by provision of a DNA construct or a vector expressing the site-directed endonuclease.

In one embodiment, the endonuclease, a guide RNA allowing to target a specified site in the gene of interest, and the nucleic acid molecule comprising the multi-stop cassette, are introduced within the cells, by the use of vectors, as genetic constructs, the protein being produced by the cell machinery (after transcription and translation) and the guide RNA and the nucleic acid molecule comprising the multi-stop cassette being transcribed by the cell machinery. Such genetic constructs can be introduced within the genome of the cells (genomic integration) or on extrachromosomal vectors (such as plasmids or artificial chromosomes). They can also be introduced as such, for instance when bombardment is performed.

The DNA constructs used in these methods can be introduced in the cells, by any method known in the art. In particular, it is possible to cite methods of direct transfer of genes such as direct micro-injection into embryos or nuclei, vacuum infiltration or electroporation, direct precipitation by means of PEG or the bombardment by gun of particles (preferably gold particles) covered with the DNA of interest. One can also introduce transgenes by protoplast transformation.

In one embodiment, it is of interest to provide the endonuclease (preferably the Cas protein) to cells present in a callus by bombardment. A vector containing a gene coding for the endonuclease, a vector containing a sequence for producing the guide RNA and the nucleic acid molecule comprising the multi-stop cassette are used for coating particles (in particular gold particles). The particles are then bombarded onto the cells. The nucleic acid molecules thus penetrate within the cells. The endonuclease (preferably the Cas protein) is thus produced, and is targeted to the site of interest *via* the guide RNA, so as to cut the genomic DNA at the site of interest. The multi-stop cassette having stop codons in all six frames can thus be integrated within the genome when the cut is repaired by NHEJ. In some embodiments, the cells already present, in their genome, a gene coding for the endonuclease (they are obtained from a plant genetically modified for the endonuclease), and only vectors containing a sequence for producing the guide RNA and the nucleic acid molecule comprising the multi-stop cassette are provided to the cells.

After bombardment, the callus can be cultured *in vitro* and a plant can be regenerated from the cultured callus. The plant will contain cells, the genome of which has incorporated the multi-stop cassette having stop codons in all six frames. Plants in which all cells have incorporated the nucleic acid molecule having stop codons in all six frames can be obtained from the seeds of the plant (in particular after auto-fecundation).

The sequence encoding the endonuclease protein and the guide RNA are under the control of any adequate promoter operative in plants (i.e. which drives transcription of the gene which it controls in plants). One can use, as an illustration, a constitutive promoter, a tissue-specific promoter (and in particular a promoter that is expressed in embryos, in pollen or in ovarian cells), or an inducible promoter. When working on plants, and although some promoters may have the same pattern of regulation when there are used in different species, it is often preferable to use monocotyledonous promoters in monocotyledons and dicotyledonous promoters in dicotyledonous plants. The promoters for endonuclease protein and the guide RNA expression may be identical or different.

Examples of constitutive promoters useful for expression include the 35S promoter or the 19S promoter (Kay et al., 1987), the rice actin promoter (McElroy et al., 1990), the pCRV promoter (Depigny-This et al., 1992), the CsVMV promoter (Verdaguer et al., 1998), the ubiquitin 1 promoter of maize (Christensen et al., 1996) and the ubiquitin promoter from rice or sugarcane.

Other promoters that can be used are the U3 promoter (*P*. *patens* U3 promoter 82) and the U6 promoter (*P*. *patens* U6 promoter; ZmU6 promoter, TaU6 promoter).

These genetic sequences shall also preferably contain any genetic elements (terminators, 5'UTR...) making it possible to obtain or optimize the expression of the nucleic acid. Such genetic elements are known in the art and can be selected by the person of skill in the art depending on the plant in which the genetic construct shall be expressed and/or the cell type in which expression is required.

The endonuclease and the guide can be cloned in a single expression cassette in a single vector or in several cassettes in the same vector or in several cassettes in several vectors.

It is preferred when the cells that are exposed to the endonuclease and the nucleic acid molecule containing the multi-stop cassette are cultured in conditions appropriate to allow chromosome replication and mitosis (the conditions are similar to that used for classical CRISPR-Cas sequence modification). Screening of the cultured cells can be performed to identify the cells in which such integration was achieved.

Screening can be performed by any method known in the art, in particular as performed for other methods of CRISPR-Cas sequence modification. One can, for instance, isolate the DNA from the part of the cultured cells and sequence the sequence of interest to verify that the nucleic acid molecule containing the stop codons was inserted at the target site. Alternatively, one can use probes appropriate to detect the desired insertion at the target site, or perform PCR amplification with appropriate primers to amplify a region around the site of interest, the insertion leading to a longer amplified sequence. Using PCR or sequencing makes also possible to quantify the number of cells (the percentage of cells among total cells) in which edition occurred if quantitative methods are used.

By way of example, one can extract the DNA of a cell, of a tissue or an organism, amplify the nucleotide sequence of interest with specific primers by PCR and sequence the sequence of interest to detect the presence of the expected modification. The sequencing can be implemented using Next Generation Sequencing (NGS). One can also use the droplet digital PCR method (ddPCR^{™} BIO RAD).

In another embodiment, it is possible to culture the cells *in vitro,* regenerate whole plants and use a plant sample from the regenerated cells to screen for the presence of the desired insertion at the target site.

This screening can make it possible to identify cells in the genome of which the nucleic acid molecule carrying the stop codons has been introduced at the target site. It can also be performed to verify that some cells of population of cells that have been exposed to the endonuclease and the nucleic acid molecule carrying the stop codons have indeed integrated such nucleic acid molecule, and/or to quantify the number of cells having integrated such nucleic acid molecule.

The invention is preferably performed on plant cells or in plant tissues, namely in cereal cells or tissues. One can cite cereals like rice, wheat, barley, sorghum, oat, maize but also sugarcane. Plant tissues can be embryos, shoot apical meristem (SAM), plant parts like pollen, microspores, leaves or plant explants. One can also use cultured cells that can be cultured as callus.

When the method is performed on plant cells, one can use the totipotency property of such plant cells, which makes it possible to regenerate a whole plant from a given cell (for instance after growing the cell and forming a callus from the cultured cells).

The invention also relates to a plant, in particular a cereal comprising, in its genome or in an extrachromosomal vector, DNA constructs herein described. The plant may thus contain, in its genome, a DNA construct coding for an endonuclease, in particular a Cas protein with the genetic elements allowing transcription in a plant cell. This construct is a transgene that have been integrated in the plant's genome by any method known in the art. In another embodiment, it is foreseen that this DNA construct is present in a vector that is extrachromosomal (not in the genome of the plant), in particular when transient expression is desired.

In an embodiment, the plant further comprises, in its genome or in an extrachromosomal vector, a guide RNA as herein defined to target the endonuclease to the desired site of the gene of interest.

The invention also relates to a plant, in particular a cereal containing, in its genome, a multi-stop cassette having six stop codons (in all six frames of readings), wherein the multistop cassette presents at most 20 nucleotides, more preferably at most 15 nucleotides, more preferably at most 14 nucleotides, most preferably at most 12 nucleotides. This means that, should a nucleotide at the 5' or the 3' end of the multistop cassette be deleted or not be taken into account, such sequence would not present the six stop codons in all six frames of readings anymore.

The invention also relates to a plant, in particular a cereal containing, in its genome, a multistop cassette having six stop codons (in all six frames of readings), in particular SEQ ID NO: 1 or SEQ ID NO: 2, or any other sequence of Table 1.

In the preferred embodiment, the method is performed in wheat, in particular in bread wheat (*Triticum aestivum,* common wheat). The invention may also be performed in *T. durum.*

It is reminded that *T. aestivum* is hexaploid (it contains the three genomes A, B and D), and that *T. durum* is tetraploid (A and B genomes).

In a preferred embodiment, the endonuclease, in particular the Cas nuclease is targeted to a site of interest on all three genomes of the wheat.

In this embodiment, it is intended to introduce the nucleic acid sequence containing stop codons in all six frames or reading in all three genomes. The site of interest is thus present in all three genomes. This means that the site of interest is present in all three genomes. For instance, this is the case when it is desired to introduce this nucleic acid molecule in the three homeologous copies of a given gene on the three genomes. It is reminded that homeologous genes are genes that are present on two different genomes, and that are partially homologous, as a result of ancestral homology.

In order to obtain such integration, it is necessary to target the endonuclease, in particular the Cas nuclease to each of the three genomes of *T. aestivum* (or of the two genomes for *T. durum*). This can be achieved either by
- select a zone that is homologous or identical in the three genomes, and provide a unique guide RNA targeting this zone, to have the endonuclease, in particular the Cas nuclease induce a cut in each of the genome
- select multiple different guide RNAs, each targeting one genome, so that the endonuclease, in particular the Cas nuclease is directed to each of the genomes, by each of the guide RNAs (this can be achieved with a guide RNA for each genome, or with a guide RNA targeting two genomes and another guide RNA targeting the last genome).

In another embodiment, the endonuclease, in particular the Cas nuclease is targeted to a site of interest that is present on two genomes of wheat, and not on the third one. This would result in the integration of the nucleic acid molecule containing the stop codons in two genomes only.

In order to obtain such integration, it is necessary to target the endonuclease, in particular the Cas nuclease to the required two genomes of *T. aestivum* and not to the third one. This can be achieved either by
- select a zone that is homologous or identical in the required two genomes, but absent on the third genome and provide a unique guide RNA targeting this zone, to have the endonuclease, in particular the Cas nuclease induce a cut in each of the required two genomes
- select multiple different guide RNAs, each targeting one required genome but not the third one, so that the endonuclease, in particular the Cas nuclease is directed to each of the required genomes, by each of the guide RNAs.

In another embodiment, the endonuclease, in particular the Cas nuclease is targeted to a site of interest that is present on one genome of wheat only, and not on the other two. This results in the integration of the nucleic acid molecule containing the stop codons in one genome only.

This can be achieved by using a guide RNA that targets a zone of the genome of interest that is not present on the other two genomes.

The invention also relates to a method for generating a cereal, comprising performing the method herein disclosed, for introducing a nucleic acid sequence containing stop codons in all six reading frames and regenerating a cereal from the resulting cells (the cells to whom the endonuclease, in particular the Cas protein and the nucleic acid sequence containing the stop codons have been provided).

In some embodiments, the cereal is regenerated from all cells to which the endonuclease, in particular the Cas protein and the nucleic acid sequence containing the stop codons have been provided. In this embodiment, the method is performed and the cereal is regenerated, for instance from a callus obtained after culture of the cells. Hence, the regenerated cereal is generally chimeric with regards to the integration of the nucleic acid sequence containing the stop codons in its genome (the genome of some cells has integrated the nucleic acid sequence containing the stop codons, whereas the genome of other cells hasn't). In this embodiment, and in order to obtain a cereal in which all cells have integrated the nucleic acid sequence containing the stop codons, one can isolate some cells and regenerate a cereal from these cells. One can also envisage self-fertilization (selfing) and screening of the progeny.

In some embodiments, the plant, in particular the cereal is regenerated from isolated cells in the genome of which the nucleic acid sequence containing the stop codons has been integrated. In this embodiment, the transformed cells are isolated. Hence, the regenerated plant contains the nucleic acid sequence containing the stop codons integrated in the genome of all of its cells.

The invention thus also relates to a plant, in particular a cereal containing a multi-stop cassette having stop codons in all six frames of reading in its genome, wherein such sequence contains at most 20 nucleotides, preferably at most 15 nucleotides, more preferably at most 14 nucleotides, most preferably at most 12 nucleotides.

In some embodiment, all cells of the cereal contain the stop cassette containing the stop codons in their genomes.

In some embodiments, the plant, in particular the cereal is homozygous for the stop cassette containing the stop codons, which is this present on both chromosomes at the target site, and in particular SEQ ID NO: 1, SEQ ID NO: 2 or any sequence of Table 1.

In preferred embodiments, the cereal is wheat, in particular bread wheat.

In these embodiments (when the cereal is wheat), one envisages when the nucleic acid sequence containing the stop codons is integrated in all three genomes of the cereal. It is also preferred when the plant is homozygous for this sequence for each genome.

In other embodiments, the nucleic acid sequence containing the stop codons is integrated in only two genomes (A, B but not D; B, D but not A, A, D but not B) of the cereal. It is also preferred when the plant is homozygous for this sequence for each genome in which it has been integrated.

In other embodiments, the nucleic acid sequence containing the stop codons is integrated in only one genome (A, B or D) of the cereal. It is also preferred when the plant is homozygous for this sequence for the genome in which it has been integrated.

The invention also relates to a nucleic acid molecule comprising SEQ ID NO: 1. The invention also relates to a nucleic acid molecule comprising SEQ ID NO: 2. The invention also relates to a nucleic acid molecule comprising SEQ ID NO: 3. The invention also relates to a nucleic acid molecule comprising SEQ ID NO: 4. The invention also relates to a nucleic acid molecule comprising SEQ ID NO: 5. The invention also relates to a nucleic acid molecule comprising SEQ ID NO: 6. The invention also relates to a nucleic acid molecule comprising SEQ ID NO: 7. The invention also relates to a nucleic acid molecule comprising SEQ ID NO: 8. The invention also relates to a nucleic acid molecule comprising SEQ ID NO: 9. The invention also relates to a nucleic acid molecule comprising SEQ ID NO: 10. The invention also relates to a nucleic acid molecule comprising SEQ ID NO: 11. The invention also relates to a nucleic acid molecule comprising SEQ ID NO: 12. The invention also relates to a nucleic acid molecule comprising SEQ ID NO: 13. The invention also relates to a nucleic acid molecule comprising SEQ ID NO: 14. The invention also relates to a nucleic acid molecule comprising SEQ ID NO: 15. The invention also relates to a nucleic acid molecule comprising SEQ ID NO: 16. The invention also relates to a nucleic acid molecule comprising SEQ ID NO: 17. The invention also relates to a nucleic acid molecule comprising SEQ ID NO: 18. The invention also relates to a nucleic acid molecule comprising SEQ ID NO: 19. The invention also relates to a nucleic acid molecule comprising SEQ ID NO: 20. The invention also relates to a nucleic acid molecule comprising SEQ ID NO: 21. The invention also relates to a nucleic acid molecule comprising SEQ ID NO: 22. The invention also relates to a nucleic acid molecule comprising SEQ ID NO: 23. The invention also relates to a nucleic acid molecule comprising SEQ ID NO: 24. The invention also relates to a nucleic acid molecule comprising SEQ ID NO: 25. The invention also relates to a nucleic acid molecule comprising SEQ ID NO: 26. The invention also relates to a nucleic acid molecule comprising SEQ ID NO: 27. The invention also relates to a nucleic acid molecule comprising SEQ ID NO: 28. The invention also relates to a nucleic acid molecule comprising SEQ ID NO: 29. The invention also relates to a nucleic acid molecule comprising SEQ ID NO: 30. The invention also relates to a nucleic acid molecule comprising SEQ ID NO: 31. The invention also relates to a nucleic acid molecule comprising SEQ ID NO: 32. The invention also relates to a nucleic acid molecule comprising SEQ ID NO: 33. The invention also relates to a nucleic acid molecule comprising SEQ ID NO: 34. The invention also relates to a nucleic acid molecule comprising SEQ ID NO: 35. The invention also relates to a nucleic acid molecule comprising SEQ ID NO: 36. The invention also relates to a nucleic acid molecule comprising SEQ ID NO: 37. The invention also relates to a nucleic acid molecule comprising SEQ ID NO: 38. The invention also relates to a nucleic acid molecule comprising SEQ ID NO: 39. The invention also relates to a nucleic acid molecule comprising SEQ ID NO: 40. The invention also relates to a nucleic acid molecule comprising SEQ ID NO: 41. The invention also relates to a nucleic acid molecule comprising SEQ ID NO: 42. The invention also relates to a nucleic acid molecule comprising SEQ ID NO: 43. The invention also relates to a nucleic acid molecule comprising SEQ ID NO: 44. The invention also relates to a nucleic acid molecule comprising SEQ ID NO: 45. The invention also relates to a nucleic acid molecule comprising SEQ ID NO: 46. The invention also relates to a nucleic acid molecule comprising SEQ ID NO: 47. The invention also relates to a nucleic acid molecule comprising SEQ ID NO: 48. The invention also relates to a nucleic acid molecule comprising SEQ ID NO: 49. The invention also relates to a nucleic acid molecule comprising SEQ ID NO: 50. The invention also relates to a nucleic acid molecule comprising SEQ ID NO: 51. The invention also relates to a nucleic acid molecule comprising SEQ ID NO: 52. The invention also relates to a nucleic acid molecule comprising SEQ ID NO: 53. The invention also relates to a nucleic acid molecule comprising SEQ ID NO: 54. The invention also relates to a nucleic acid molecule comprising SEQ ID NO: 55. The invention also relates to a nucleic acid molecule comprising SEQ ID NO: 56. The invention also relates to a nucleic acid molecule comprising SEQ ID NO: 57. The invention also relates to a nucleic acid molecule comprising SEQ ID NO: 58. The invention also relates to a nucleic acid molecule comprising SEQ ID NO: 59. The invention also relates to a nucleic acid molecule comprising SEQ ID NO: 60. The invention also relates to a nucleic acid molecule comprising SEQ ID NO: 61. The invention also relates to a nucleic acid molecule comprising SEQ ID NO: 62. The invention also relates to a nucleic acid molecule comprising SEQ ID NO: 63. The invention also relates to a nucleic acid molecule comprising SEQ ID NO: 64. The invention also relates to a nucleic acid molecule consisting of SEQ ID NO: 1. The invention also relates to a nucleic acid molecule consisting of SEQ ID NO: 2. The invention also relates to a nucleic acid molecule consisting of SEQ ID NO: 3. The invention also relates to a nucleic acid molecule consisting of SEQ ID NO: 4. The invention also relates to a nucleic acid molecule consisting of SEQ ID NO: 5. The invention also relates to a nucleic acid molecule consisting of SEQ ID NO: 6. The invention also relates to a nucleic acid molecule consisting of SEQ ID NO: 7. The invention also relates to a nucleic acid molecule consisting of SEQ ID NO: 8. The invention also relates to a nucleic acid molecule consisting of SEQ ID NO: 9. The invention also relates to a nucleic acid molecule consisting of SEQ ID NO: 10. The invention also relates to a nucleic acid molecule consisting of SEQ ID NO: 11. The invention also relates to a nucleic acid molecule consisting of SEQ ID NO: 12. The invention also relates to a nucleic acid molecule consisting of SEQ ID NO: 13. The invention also relates to a nucleic acid molecule consisting of SEQ ID NO: 14. The invention also relates to a nucleic acid molecule consisting of SEQ ID NO: 15. The invention also relates to a nucleic acid molecule consisting of SEQ ID NO: 16. The invention also relates to a nucleic acid molecule consisting of SEQ ID NO: 17. The invention also relates to a nucleic acid molecule consisting of SEQ ID NO: 18. The invention also relates to a nucleic acid molecule consisting of SEQ ID NO: 19. The invention also relates to a nucleic acid molecule consisting of SEQ ID NO: 20. The invention also relates to a nucleic acid molecule consisting of SEQ ID NO: 21. The invention also relates to a nucleic acid molecule consisting of SEQ ID NO: 22. The invention also relates to a nucleic acid molecule consisting of SEQ ID NO: 23. The invention also relates to a nucleic acid molecule consisting of SEQ ID NO: 24. The invention also relates to a nucleic acid molecule consisting of SEQ ID NO: 25. The invention also relates to a nucleic acid molecule consisting of SEQ ID NO: 26. The invention also relates to a nucleic acid molecule consisting of SEQ ID NO: 27. The invention also relates to a nucleic acid molecule consisting of SEQ ID NO: 28. The invention also relates to a nucleic acid molecule consisting of SEQ ID NO: 29. The invention also relates to a nucleic acid molecule consisting of SEQ ID NO: 30. The invention also relates to a nucleic acid molecule consisting of SEQ ID NO: 31. The invention also relates to a nucleic acid molecule consisting of SEQ ID NO: 32. The invention also relates to a nucleic acid molecule consisting of SEQ ID NO: 33. The invention also relates to a nucleic acid molecule consisting of SEQ ID NO: 34. The invention also relates to a nucleic acid molecule consisting of SEQ ID NO: 35. The invention also relates to a nucleic acid molecule consisting of SEQ ID NO: 36. The invention also relates to a nucleic acid molecule consisting of SEQ ID NO: 37. The invention also relates to a nucleic acid molecule consisting of SEQ ID NO: 38. The invention also relates to a nucleic acid molecule consisting of SEQ ID NO: 39. The invention also relates to a nucleic acid molecule consisting of SEQ ID NO: 40. The invention also relates to a nucleic acid molecule consisting of SEQ ID NO: 41. The invention also relates to a nucleic acid molecule consisting of SEQ ID NO: 42. The invention also relates to a nucleic acid molecule consisting of SEQ ID NO: 43. The invention also relates to a nucleic acid molecule consisting of SEQ ID NO: 44. The invention also relates to a nucleic acid molecule consisting of SEQ ID NO: 45. The invention also relates to a nucleic acid molecule consisting of SEQ ID NO: 46. The invention also relates to a nucleic acid molecule consisting of SEQ ID NO: 47. The invention also relates to a nucleic acid molecule consisting of SEQ ID NO: 48. The invention also relates to a nucleic acid molecule consisting of SEQ ID NO: 49. The invention also relates to a nucleic acid molecule consisting of SEQ ID NO: 50. The invention also relates to a nucleic acid molecule consisting of SEQ ID NO: 51. The invention also relates to a nucleic acid molecule consisting of SEQ ID NO: 52. The invention also relates to a nucleic acid molecule consisting of SEQ ID NO: 53. The invention also relates to a nucleic acid molecule consisting of SEQ ID NO: 54. The invention also relates to a nucleic acid molecule consisting of SEQ ID NO: 55. The invention also relates to a nucleic acid molecule consisting of SEQ ID NO: 56. The invention also relates to a nucleic acid molecule consisting of SEQ ID NO: 57. The invention also relates to a nucleic acid molecule consisting of SEQ ID NO: 58. The invention also relates to a nucleic acid molecule consisting of SEQ ID NO: 59. The invention also relates to a nucleic acid molecule consisting of SEQ ID NO: 60. The invention also relates to a nucleic acid molecule consisting of SEQ ID NO: 61. The invention also relates to a nucleic acid molecule consisting of SEQ ID NO: 62. The invention also relates to a nucleic acid molecule consisting of SEQ ID NO: 63. The invention also relates to a nucleic acid molecule consisting of SEQ ID NO: 64.

The invention also relates to a multistop cassette, comprising one of SEQ ID N°1 to SEQ ID N°64, flanked by 5 to 20 nucleotides at each extremity. This cassette is preferably a linear DNA or RNA fragment.

The methods herein disclosed are or particular interest for evaluating the role of a gene of interest in a plant, notably a cereal. Such methods shall comprise
(a) Performing a method herein disclosed, to introduce the nucleic acid containing the stop codons within the gene of interest
(b) Regenerating a plant comprising the nucleic acid containing the stop codons introduced within the gene of interest,
(c) Optionally obtaining a plant homozygous for the nucleic acid containing the stop codons introduced within the gene of interest
(d) Comparing the phenotype of the plant of (b) or (c) with the phenotype of an isogenic plant not having the nucleic acid containing the stop codons introduced within the gene of interest
thereby determining the role of the gene of interest as inducing the difference of the phenotypes compared in (d). This comparison of (d) is performed with plants observed/grown in the same conditions. The phenotype can thus be determined for the conditions in which the plants have been grown/observed (i.e. the conditions in which the phenotypes have been compared).

Two isogenic or near-isogenic plants are plants for which the genome are almost identical (having the same or closely similar genotypes), except for the locus at which the nucleic acid containing the stop codons has been introduced.

Such plants can be obtained by methods known in the art, notably by backcrosses to introduce the locus with the nucleic acid containing the stop codons into a reference elite line, so that the plant resulting from the backcrosses (and after selfing) will be isogenic or nearly-isogenic to the elite line.

### FIGURES

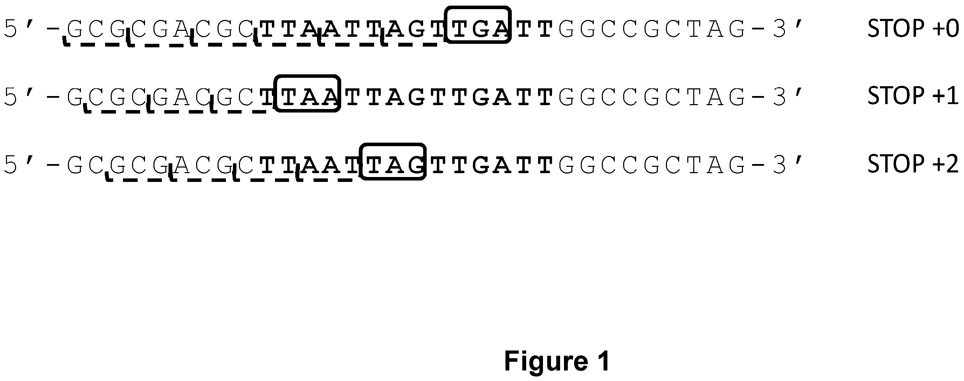

Figure 1: a: 3-stop cassette (SEQ ID NO: 68); in bold, the 14nt core containing the stop codons, flanked by its borders showing the 3 possible reading-frames in which the cassette might end after genomic insertion, the reading-frames are underlined, the first stop encountered is framed.

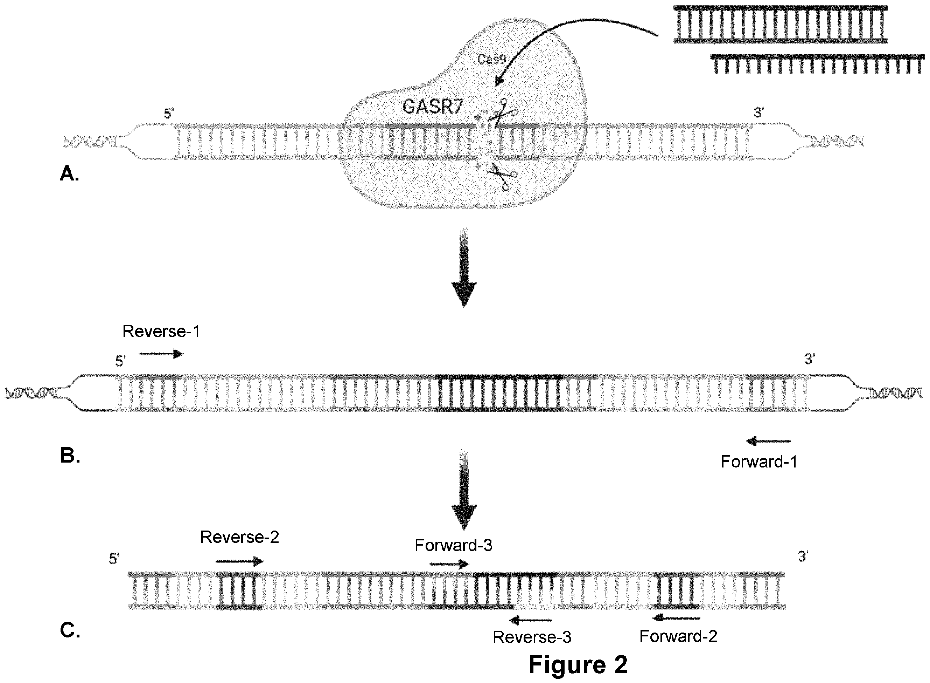

Figure 2: schematic representation of an embodiment of the process. A. The target chromosome is cut by Cas9 at a locus of interest (here GASR7 locus) and a multi-top cassette (double-stranded or single stranded) is provided. B. A first PCR with primers Forward-1 and Reverse-1 is performed to amplify the locus of interest. C. A second and a third PCR are performed with Forward-2 or 3 and Reverse-2 or 3 primers.

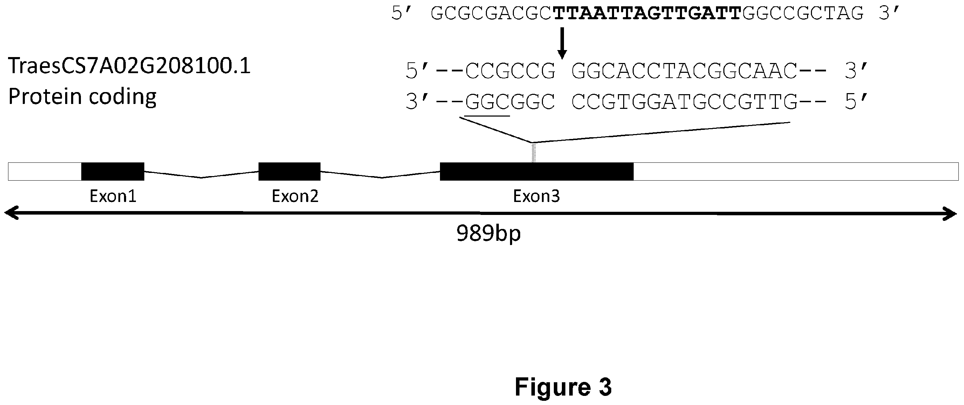

Figure 3: targeted insertion of the 3-Stop cassette (SEQ ID NO: 68) at the GASR7 locus (SEQ ID NO: 69 / SEQ ID NO: 83). Schematic diagram of TaGASR7, with 5' and 3' UTR as white boxes, exons as black boxes, introns as lines, the PAM of the targeted sequence underlined, the position of cassette insertion in the third exon as a grey bar, and the cassette with its 14nt core in bold.

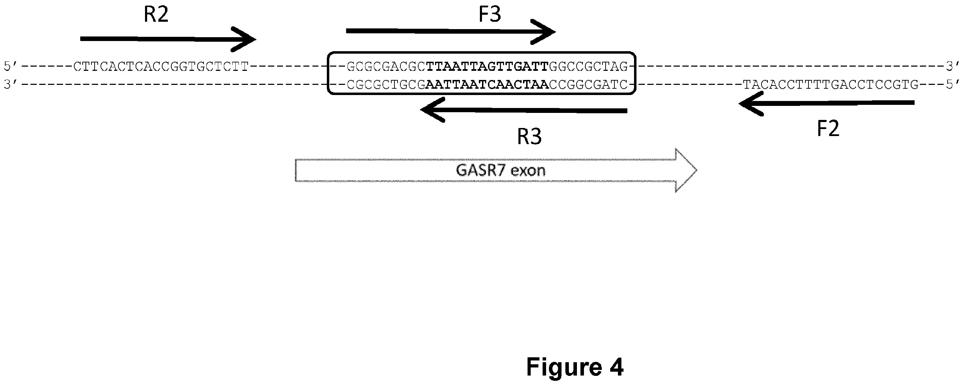

Figure 4: schematic representation of the 2nd step of the nested PCR/The cassette specific amplification on the B genome. The 3-stop cassette is boxed, with the 14nt core in bold; each primer is represented by an arrow. F2 (SEQ ID NO: 75) and R2 (SEQ ID NO: 76) are genome specific primers distal to the insertion site, F3 (SEQ ID NO: 81) and R3 (SEQ ID NO: 82) are specific of the 3-stop cassette.

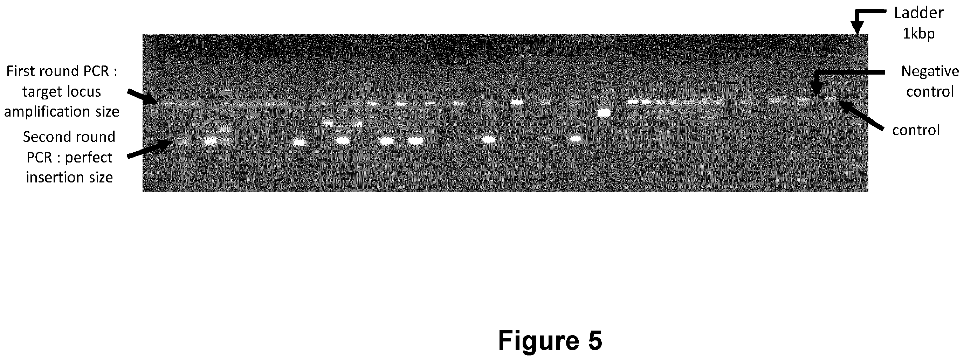

Figure 5: Representative example of nested PCR results. This gel is a result from the second step of the nested PCR. The bands at 612bp are residual fragments of the first amplification. The second amplification, specific for cassette insertion, should show a band at 294bp for a perfect insertion. Some bands are higher that 294bp, meaning a larger insertion than the sole cassette, and multiple bands can be seen from a single well, meaning a heterozygous or multi-insertions event. Negative control is the PCR control without DNA, Control is a modality shot without the 3-stop cassette 612bp corresponding to amplification of the loci without insertion for genome B.

### EXAMPLES

### Example 1 CRISPR-Cas9 indels at GASR7 locus can generate the translation of long, unknown proteins

The most straightforward method of KO induction using the CRISPR-Cas9 system is the generation of small indels for frameshift disruption. A canonical Cas9-generated indel can create two types of frameshifts, for +1bp or +2bp indels (equivalent to -2bp and -1bp respectively in term of frameshift). So, the new protein sequence resulting from these frameshifts can be predicted. The TaGASR7 proteins targeted in this study are 101aa, 99aa, 94aa long on the A, B and D genomes respectively. With the GASR7 gRNA used (SEQ ID NO: 70), targeting SEQ ID NO: 69), when inducing a +1bp frameshift, the protein sequence extends by 28aa and 3aa on the A genome and B or D genome respectively. When inducing a +2bp frameshift, the protein sequence extends by 185aa, 32aa and 108aa on the A, B and D genome respectively. So, a classical Cas9-mediated genome editing would produce a new protein that can be around 3%, 30%, 100% or 180% longer.

A way to avoid these potential long and unknown newly generated proteins would be to generate precisely and with certainty a stop codon at the Cas9-generated mutation position. A "multi-stop" cassette was designed to fulfill this goal. This "multi-stop" cassette can be designed such that Stops occur in all 3 reading frames of one direction of insertion, "3-Stop" cassette, or in all reading frames independent of the direction of insertion when a "6-Stop" cassette is used.

### Example 2. Design of a "3-Stop" cassette.

The 3-stop cassette is constituted of a 14 nucleotides core, containing each one of the three stop codons in one reading direction. These stop codons are arranged in such a way that at least one stop codon is read for each of the three possible reading frames (Figure 1).

The insertion of this cassette in the forward, 5' to 3' direction (direct insertion) in any coding sequence will generate a stop codon precisely at the position of insertion, as long as the 14 nucleotides core is intactly inserted.

Two stretches of nine nucleotides are contiguous to this core, one at its 5' end and one at its 3' end. They will be referred as the cassette borders. These borders were designed to provide a protection from degradations that can occur in the cell prior to the cassette insertion, and to allow the design of PCR primers for specific detection of cassettes insertions.

### Example 3. Detection of stable, targeted insertions of the 3-Stop cassette in to TaGASR7 after DNA transfer into calli

In brief, the experimentation consists first in the coating of gold particles by two plasmids, SpCas9 (SEQ ID NO: 67) and an sgRNA (SEQ ID NO: 70) plasmid targeting the GASR7 gene (Figure 3), as well as the synthetic 3-Stop cassette flanked by nucleotides (SEQ ID NO: 68), either in the form of a single-strand (ssDNA) or a double-strand (dsDNA). These particles were then bombarded into immature wheat embryos using a biolistic procedure. The embryos were subsequently regenerated *in vitro* for three weeks without selection. The resulting calli were then submitted to a nested PCR to detect the insertion events. The PCR products were subsequently subjected to Sanger sequencing for analysis, and a selection was submitted to NGS for more complete analysis.

### Detection principle: nested PCR

During biolistic protocol for transformation, the gold particles probably penetrate only the upper cellular layers of the embryos. So the 3-Stop insertion cassette, Cas9 and gRNA plasmids can only be delivered to a minority of cells, whilst most of the cells of the embryos are untouched. Consequently, in a pool of callus cells cultivated without selection pressure, the number of insertion events is necessary very low compared to wild type cells (without insertion).

Thus, to obtain data on putative cassette insertion, a PCR was performed using couples of genome-specific (F2 (SEQ ID. N° 73, 75, 79) and R2 (SEQ ID. 74, 76, 80)) and cassette-specific primers, (F3 (SEQ ID N° 81) and R3, SEQ ID N° 82). However, no amplification signals were obtained, indicating that the target was present at very low concentrations or absent. So, nested PCRs were performed to detect cassette insertions (Figures 2 and 4). In the nested PCR, the first amplification using 3 pairs of genome-specific Forward 1 (SEQ ID. N° 71, 75, 77) and Reverse 1 primers (SEQ ID. N° 72, 76, 78) amplifies the target locus of the sequence of interest from each genome, in order to enrich for the DNA portion potentially containing the cassette insertion. The second amplification, using R2-R3 primers for 5' direct insertion border characterization, or F3-F2 primers for 3' insertion border characterization, is specific to the cassette, allowing the detection of the cassette insertion in a 5' to 3' direction from the previously enriched mix (Figure 4). Second amplifications with F2-R3 and F3-R2 primer pairs allowed detection of the cassette when integrated into GASR7 in the reverse orientation.

### Detection of insertion events

From the 391 embryos bombarded and pooled into two embryos per sample before DNA extraction, 197 events of insertion were detected in the 5' to 3' Direct orientation and 177 in the Reverse orientation.

On agarose gels, a perfect insertion would be characterized by a single band at the expected size after the second round of PCR. The results showed events characterized by a single band at the expected height, a single band lower or higher than the expected position, multiple bands around the expected position or lower or higher (see Figure 5 for an example of bands higher than the expected position). This means that the cassette was integrated into the wheat genome either perfectly as designed *in silico,* but also with genomic DNA insertions or deletions ranging from few tens to few hundred nucleotides, and that the cassette itself might have been truncated during the process of insertion.

To conclude, insertion events can be detected in a pool of calli even with a small proportion of cells reached due to the transformation method. Nonetheless, the hundreds of detected events show that when cells are reached, the insertion event is not rare in calli.

### The ssDNA cassette is more efficient than a dsDNA cassette to generate insertion events

145 immature embryos were bombarded with the single-strand cassette (ssDNA), and 246 with the double-strand cassette (dsDNA), all other experimental conditions being equal. The frequency of cassettes insertion was higher for the ssDNA modality, with 253 events against 120 events for the dsDNA modality. From these PCR detections, it seems that the insertion into the wheat genome using the single-strand DNA is about four times more frequent than when double-strand DNA is used.

### Insertions are detected in all three wheat genomes

Considering all conditions (Direct/Reverse and ssDNA/dsDNA); 189 insertions were detected in the B genome, 84 in the A genome and 101 in the D genome.

### Sequencing data indicates that insertions occurred systematically at the expected position, in both senses of orientation and sometimes more than one copy is inserted

Sanger sequencing of amplicons from the second step of the nested PCR was performed, using R2-R3 primers for 5' direct insertions border characterization or F3-F2 primers for 3' reverse insertion border characterization.

In all the studied events, cassette insertions occurred at the Cas9 cleavage position. This means that the insertions were consecutive to the Cas9 cleavage, and not random insertions.

### Characterization of Insertion events.

The cassette was observed in 82 % of the "studied events". As stated above, an observation here means that the cassette was inserted with at least a part of its borders intact. The remaining 18% are events for which a cassette insertion occurred, but whose edges have been so truncated that they are no longer visible (hybridization of R3 or F3 is no longer possible due to truncation of the cassette, so no amplification occurs). Among 82% of the observations, the cassette was truncated 94% of the time, with a mean of 5bp truncated.

In 46% of the studied events, a genomic deletion also occurred, with a mean of 3bp on the 5'end.

Other types of insertions were also characterized, for example canonical Cas9 insertions, cassette concatenations, plasmid parts or (rare) unknown insertions. All together, these insertions occurred 23% of the time on the 5' end of the cassette, with an average insertion of 17bp. They occurred 91% of the time on the 3' end of the cassette, with an average insertion of 86bp. Among this last result, at least 33% are multiple cassettes inserted end-to-end.

Among all the studied events, only one was characterized has perfect, meaning the sole insertion of one intact cassette without any genomic indel. This event is located on both copies of the B genome. The unpredictability of the type of cassette insertion and the potential surrounding genomic changes seen in these results, confirms that with such a simple system of cassette insertion, an insertion in phase with a desired reading frame is not easily obtainable. This emphasizes the importance of having stop codons in all the forward reading-frames of the cassette to ensure translational termination irrespective of the nature of the insertion of the cassette assuming that the cassette core is inserted.

### NGS identification of insertion events indicates that the extended borders were efficient for protection of the core stretch of the cassette.

The limit of Sanger sequencing, originating from the limitation of using a specific primer that binds to one side of the cassette is the impossibility to have a complete sequence read over one event as the primers necessarily covers a part of the cassette. Importantly, this means that if a heavily truncated cassette was inserted, then no amplification would be seen. In other words, the nested PCR method described above implied a counter-selection of non-functional cassette insertions, or of cassettes that lacks the borders even if they are functional.

To overcome this blind spot, a NGS (Next Generation Sequencing) analysis was performed on five selected pools of calli, genomic specifically (so that the amplicons can be distinguished according to their genomic origin) and with a read depth sequencing ranging from 1 to 1,5 million reads per sample.

The amplicons were produced by a PCR amplification strategy. Firstly, the PCR uses specific primers to amplify the B-genome TaGASR7 (target sequences 5' GTTTGTGCAGAGTGCTCGTC 3' (SEQ ID NO: 84) and 5' TTAAATGCCCATCACATAATTCA 3' (SEQ ID NO: 85)) which are extended by universal tags. Secondly, the PCR uses as matrix the B-genome TaGASR7 and primers that will allow to produce an amplicon with functional P5 and P7 Illumina adapters. A double index barcoding system is also included for identifying samples after sequencing. The sequencing was performed on a MiSeq (Illumina) with a paired end strategy of 2 reads of 250 bases. The expected coverage per amplicon is 1 million reads. Sequences were analyzed using Geneious Prime 2022.0.1 (https://www.geneious.com).

First, among the five samples, each one being a pool of calli, four cassette insertion events were characterized. In all four of these events, the observed cassettes were intact for their 14bp core, meaning that the insertions are operational.

In details, for the ssDNA modality, one sample was submitted to NGS. From the previous Sanger analysis, this sample was characterized by a truncation of 5bp of the cassette border in 3' and by the deletion of one genomic base in 3'. No information was available on its 5' side because of an indistinct signal. Thanks to NGS, two independent insertions events were characterized in this sample. The first one consisted of the insertion of one cassette of 19bp (1bp of the 5' border + the 14bp core + 4bp of the 3' border) followed in 3' by 6bp of undefined origin and followed a second partial cassette of 12bp (8bp of the core + 4bp of the 3' border) in a reverse orientation, for a 37bp insertion in total. This characterization is perfectly coherent with the Sanger analysis. This event was observed in 0,033% of the reads. The second one consisted of the insertion of one cassette truncated by 2bp on the 5' and 6bp on the 3', followed in 3' by the insertion of 71bp of the Cas9 plasmid, for a 95bp total insertion. This event was observed in 0,014% of the reads.

For the dsDNA modality, one sample was submitted to NGS, which was previously characterized from Sanger sequencing by a truncation of 2bp of the cassette border in 5' and an indistinct signal in 3'. Thanks to NGS, two insertions were also characterized in this sample. The first one consisted of the insertion of two adjacent cassettes, the first being truncated of 2bp in 5' and 5bp in 3' and the second of 6bp both in 5' and 3' ends, followed by an unknown sequence of 61bp for a total insertion of 106bp. Once again, this result is coherent with the Sanger sequencing. The second one consisted of the insertion of a cassette truncated of 3bp on both ends, for 26bp in total. Both appeared at a 0,0002% rate.

| Modality | Insertion frequency (%) | Insertion size (bp) | Operational cassette |
|---|---|---|---|
| ssDNA | 0,033 | 37 | Yes |
| | 0,014 | 95 | Yes |
| dsDNA | 0,0002 | 106 | Yes |
| | 0,0002 | 26 | Yes |

**Table 2: Summary table of the 3-Stop cassette insertion events characterized by NGS.** Two events were characterized for each modality, namely ssDNA for the cassette shot under the single-strand form and dsDNA for the cassette shot under the double-strand form. The frequency of the sequences containing an insertion is indicated in % relative to all the reads, after normalisation of the background noise, and their corresponding size is indicated in number of bp. The 3-Stop cassette is operational in all of these events.

In three out of the five samples, no cassette was detected. This is probably due to a too shallow depth of sequencing, as the low frequency of cassette detections for the other analyses suggests.

For the ssDNA sample, with two insertions representing 0,047% of the reads (0,014% + 0,033%), the total proportion of indels is 0,251% at the canonical position of insertion (3bp after the PAM, the position of the cassette insertions). Compared to the 0,047% of cassette insertions in total, this means that the cassette was inserted around 5 times less than the occurrence of indels at the canonical Cas9 mutation position. This suggests that the insertion of a small cassette thanks to biolistic in plants regenerated without selection is achievable thanks to an experimental design with five time more embryos than the usual Cas9 experiment.

### Stable insertion of the 3-Stop cassette into Wheat

Following the previous results, an insertion experiment was performed until the adult plant stage. The 3-Stop cassette was transfected by the same method (biolistics) under the ssDNA form only as this was the most efficient modality in the callus experiment, on 4774 embryos. In addition to the ssDNA 3-Stop cassette and the SpCas9 and gRNA plasmids, a plasmid encoding PMI for plant selection was also included in the bombardment. The plantlets were regenerated following PMI selection (Wright et al., 2001).

The mutations and/or insertions of the cassette were detected in the regenerated T0 plants using a PCR restriction enzyme (PCR-RE) assay (Zhang et al, Nat Commun 7, 12617; 2016) with three pairs of genome-specific Forward 1 (SEQ ID. N° 71, 75, 77) and Reverse 1 primers (SEQ ID. N° 72, 76, 78) each specific to a TaGASR7 homeologue. The insertion frequency is defined as the number of plants carrying the cassette insertion over the number of embryos shot.

A cassette insertion was detected in one plant, which was submitted to Sanger sequencing. The insertion occurred in the B genome, at the Cas9 cleavage site. The cassette was truncated to 17bp, with the core 14bp intact, and 2bp of the 5' border and 1bp of the 3' border remaining. A deletion of 88bp of the genomic DNA was present, along with unidentified insertions of 6bp and 2bp in 5' and 3' of the cassette respectively.

First, this result proves that it is achievable to regenerate a plant containing the multistop cassette, and thus that the multi-STOP cassette insertion in the wheat genome is feasible for an applicative standpoint.

Moreover, the insertion profile seems to be similar to the one observed in the callus experiment, with indels in the genomic DNA and degradation of the cassette. This emphasizes the usefulness of the multi-STOP design.

### Example 4: Insertion of a "6-Stop cassette" into wheat GASR7.

Since insertion of the Stop cassette can be either in a direct or reverse orientation it is desirable that both directions of insertion induce Stops in all reading frames. This can be achieved by designing a 6-Stop cassette, potential designs are which are shown in Table 1. A variant of these designs including SEQ ID NO 1 (SEQ ID NO 65) was co-transformed into wheat with pPMI, SpCas9, GASR7gRNA, as either ssDNA or dsDNA, as described in Example 2. Plants are regenerated and identified by PCR and DNA sequencing for insertion of the "6-Stop" cassette in the direct or reverse orientation.

### Example 5. Material and methods

### Plant material

*Triticum aestivum* cv. Bobwhite S26 were grown in a greenhouse under controlled conditions with day temperatures of 18°c for 16h and night temperature of 15°c for 8 hours.

### Multistop 3-Stop and 6-Stop cassette preparation

The cassette was synthetized by Sigma Aldritch in standard conditions in the form of a single-strand (ssDNA). The cassette was synthetized by ProteoGenix under the form of a double-strand (dsDNA). In both cases, it was diluted to 50pmol/µl prior to the transfection.
3-Stop dsDNA cassette with its borders:
   5'-GCGCGACGCTTAATTAGTTGATTGGCCGCTAG-3' (SEQ ID NO: 68)
6-Stop dsDNA cassette with its borders :
   5'-CGTGCCGCCGTTAATTAGTTAACTAAGGCACCTACG-3' (SEQ ID NO: 65)

### Plasmids

### Construction of the SpCas9 plasmid

The Cas9 rice optimized gene was put under a ZmUbi promoter (Christensen et al. 1992)

### Construction of the guide RNA plasmid

The 18bp guide sequence, targeting the third exon of the GASR7 gene, was taken from Zhang et al. (2016) and was put under an U6 promoter codon optimized for wheat (Shan et al. 2014). To ensure good U6 polymerase III expression, the guide sequence contain a G at its 5' end. This synthetic construct was inserted inside a pUC57 backbone by Life technologies.

### PMI plasmid

A fragment of pNOV2820 containing the E-coli-derived *manA* gene (also named *pmi* gene in Wright et al. (2001)) encoding PMI, has been placed under a maize ubiquitin intron 1 promoter and a nos terminator in pAHC17 (Christensen and Quail, 1996).

### Biolistic transformation and wheat regeneration

Immature seeds were harvested fourteen days post-anthesis, sterilized for 20min in a 20% hypochlorite solution containing 1 drop per 200mL of Teepol. Immatures embryos were isolated for bombardment under binocular magnifier in a sterile environment, after zygotic meristem removal to prevent precocious germination, at a rate of fifty embryos per medium plate. Isolated embryos were placed on plasmolysis medium for 4h before bombardment. As a control, fifty additional embryos from the same cultivation conditions were transferred to the osmotic medium without bombardment. The transformation was performed using a PDS1000/He particle bombardment system (Bio-Rad) with a target distance of 6-cm from the stopping plate with a helium pressure of 900 PSI. 20-µl of gold microparticles solution (50mg.ml⁻¹, 0,6µm diameter, Bio-Rad) were sonicated for 1min and suspended in a DNA solution containing 0,3pmol of each plasmid and 100pmol of the cassette, the cassette being in excess to increase the probability of insertion. Next, 20 µL of 0.1 M spermidine and 50 µL of 2.5 M CaCl2 were mixed with the suspension and incubated for 15 min at room temperature under gentle agitation and then centrifuged for 1 min. The supernatant was removed, and after being washed in 99.8% ethanol, the gold bullets were re-suspended in 100 µL of ethanol. For each bombardment, 10µL of microparticle DNA were placed on the microcarrier.

The biolistic and wheat regeneration method follows the protocol from Pellegrineschi et al., 2002. The exception concerns the calli experiment, in which the embryos were placed on MSE3 medium (Pellegrineschi et al., 2002) the day after bombardment and cultivated in a dark chamber at 32°c for three weeks, after which the DNA of the induced calli was extracted. In the adult plant experiment, the selection of PMI transgenic events was performed as described by Wright et al. 2001.

### DNA extraction

Calli were collected in Eppendorf SafeLock tubes (2mL, 2 calli per tube), in which an autoclaved 5mm stainless steel bead was previously added. The tubes were then immersed in liquid nitrogen. Grinding was performed for two rounds of 30s at 1200rpm using Qiagen TissueLyser II (Qiagen). After grinding, 350µl of ambient temperature extraction buffer was added to each tube. DNA extraction was done using the cetylmetylammonium bromide (CTAB) method from Tassy et al. 2006.

### Nested PCR

Multistop cassette insertions were detected by nested PCR using first genome specific primers F1 and R1, followed by a second amplification of the previous PCR product using one genomic primer, F2 or R2, and one cassette-internal primer, F3 or R3. The first reaction was carried out as follow: 25µL of PCR mix containing 12,5µL of Taq Gold Polymerase (Qiagen), 1µL of GC enhancer (Qiagen), 7.5pmol of each primer, 5µL of genomic DNA normalised at 10ng/µL and 4.5µL of ultrapure water. PCR was performed using an ABI Veriti thermocycler (Applied Biosystems) with a first step at 95°c for 10 minutes, then 35 cycles at 95°c for 30 seconds, at 60°c for 30 seconds, at 72°c for 1 minute, and a final extension step at 72°c for 1 minute. Nested PCR was performed following identical conditions, except for the 5µL of genomic DNA replaced with 5µL of the first PCR product. PCR products were submitted to agarose gel electrophoresis (2% in 1X TAE buffer).

### PCR products Sanger sequencing and sequences analysis

Sequencing reactions were performed by Eurofins Genomics, and sequences were analyzed using the SnapGene software (Dotmatics, Boston MA, USA).

### Next Generation Sequencing (NGS)

The amplicons were produced by a PCR amplification strategy. Firstly, the PCR uses specific primers to amplify the B-genome TaGASR7 (target sequences 5' GTTTGTGCAGAGTGCTCGTC 3' (SEQ ID NO: 84) and 5' TTAAATGCCCATCACATAATTCA 3' (SEQ ID NO: 85)) which are extended by universal tags. Secondly, the PCR uses as matrix the B-genome TaGASR7 and primers that will allow to produce an amplicon with functional P5 and P7 Illumina adapters. A double index barcoding system is also included for identifying samples after sequencing. The sequencing was done on a MiSeq (Illumina) with a paired end strategy of 2 reads of 250 bases. The expected coverage per amplicon is 1 million reads.

Sequences were analysed using Geneious Prime 2022.0.1 (Dotmatics, Boston MA, USA).

### List of primers

**Table 3. List of primers**

| Target | Fonction | Name | Sequence | SEQ ID NO |
|---|---|---|---|---|
| GASR7-A | Forward_1 | GASR7_A_F1 | TGGGAGAGCAGTGAATAGCC | 71 |
| | Reverse_1 | GASR7_A_R2 | GTGTGTGCAGAGTGCTCGTC | 72 |
| | Forward_2 | GASR7_A_F2 | GAATAGCCATCAACCGCAAG | 73 |
| | Reverse_2 | GASR7_A_R1 | AGAGTGCTCGTCCAAGTGCT | 74 |
| GASR7-B | Forward_1 | GASR7_B_ F2 | GTGCCTCCAGTTTTCCACAT | 75 |
| | Reverse_1 | GASR7_B_ R2 | CTTCACTCACCGGTGCTCTT | 76 |
| | Forward_2 | GASR7_B_ F2 | GTGCCTCCAGTTTTCCACAT | 75 |
| | Reverse_2 | GASR7_B_ R2 | CTTCACTCACCGGTGCTCTT | 76 |
| GASR7-D | Forward_1 | GAT 307 | CCTCCATTTTTCCACATCTTA GTCC | 77 |
| | Reverse_1 | GAT 304 | CCTTCATCCTTCAGCCATGC AT | 78 |
| | Forward_2 | GASR7_D_F1 | AAGCTGGCACCCCAGAAG | 79 |
| | Reverse_2 | GASR7_D_R1 | GTTTTTGTGTCGGTCTGTGC | 80 |
| Multi-STOP | Forward_3 | PCRe_Int_F3 | GCGCGACGCTTAATTAGTTG ATT | 81 |
| | Reverse_3 | PCRe_Int_R3 | CTAGCGGCCAATCAACTAAT TAA | 82 |
| Amplif B genome TaGAS R7 | Forward | | GTTTGTGCAGAGTGCTCGTC | 84 |
| | Reverse | | TTAAATGCCCATCACATAATT CA | 85 |

**Table 4. List of PCRs and expected products**

| Forward Primer | Revers Primer | Target | Size of amplicon |
|---|---|---|---|
| GASR7_A_F1 | GASR7_A_R2 | GASR7-A | 324 |
| GASR7_B_F2 | GASR7_B_R2 | GASR7-B | 612 |
| GAT-307 | GAT-304 | GASR7-D | 614 |
| GASR7_A_F2 | PCRe_Int_R3 | GASR7-A | 196 |
| GASR7_B_F2 | PCRe_Int_R3 | GASR7-B | 294 |
| GASR7_D_F1 | PCRe_Int_R3 | GASR7-D | 150 |
| PCRe_Int_F3 | GASR7_A_R2 | GASR7-A | 147 |
| PCRe_Int_F3 | GASR7_B_R2 | GASR7-B | 350 |
| PCRe_Int_F3 | GAT 304 | GASR7-D | 357 |
| GASR7_A_F2 | PCRe_Int_F3 | GASR7-A | 196 |
| GASR7_B_F2 | PCRe_Int_F3 | GASR7-B | 294 |
| GASR7_D_F1 | PCRe_Int_F3 | GASR7-D | 150 |
| PCRe_Int_R4 | GASR7_A_R2 | GASR7-A | 147 |
| PCRe_Int_R4 | GASR7_B_R2 | GASR7-B | 350 |
| PCRe_Int_R4 | GAT 304 | GASR7-D | 357 |

### REFERENCES

Anzalone et al. 2019. Nature 576, 149-157. doi.org/10.1038/s41586-019-1711-4 Christensen AH, Quail PH. (1996) Transgenic Res. May;5(3):213-8.
Christensen et al. 1992. Plant Mol Biol. Feb;18(4):675-89. doi: 10.1007/BF00020010
Christensen et al., 1996. Transgenic Res.5(3):213-8. doi: 10.1007/BF01969712. Depigny-This D, et al. (1992), Plant Mol Biol.;20(3):467-79.
Dong and Ronald 2021. Proc. Natl. Acad. Sci. U.S.A. 118, e2004834117. doi.org/10.1073/pnas.2004834117
Gorbunova et al., 1997. Nucleic Acids Research 25, 4650-4657. doi.org/10.1093/nar/25.22.4650
Hu et al, Nature. 2018 Apr 5;556(7699):57-63)
Itzkovitz & Alon 2007. Genome Res. 17, 405-412. doi.org/10.1101/gr.5987307
Jiang et al. 2019. Plant Mol Biol 100, 467-479. doi.org/10.1007/s11103-019-00871-5
Kay et al. (1987), Science, 236 :1299-1302
Kuscu et al. 2017. Nat Methods 14, 710-712. doi.org/10.1038/nmeth.4327
Li et al. 2016. Nature Plants 2, 16139. doi.org/10.1038/nplants.2016.139
Li etal. 2018. Genome Biol 19, 59. doi.org/10.1186/s13059-018-1443-z
Lin et al. 2020. Nat Biotechnol 38, 582-585. doi.org/10.1038/s41587-020-0455-x McElroy,D. Zhang,W. Cao,J. Wu,R. (1990) Plant Cell 2(2):163
Molla et al. 2021. Nat. Plants 7, 1166-1187. doi.org/10.1038/s41477-021-00991-1 Pellegrineschi et al. 2002. Genome 45, 421-430. doi.org/10.1139/g01-154
Puchta 2004. Journal of Experimental Botany. doi.org/10.1093/jxb/eri025
Ren et al, Molecular Plants, 12 (7), 2019, 1015-1026
Shan et al. (2013). Nat Biotechnol 31, 686-688. doi.org/10.1038/nbt.2650
Shan et al. (2014). Nat Protoc. 2014 Oct;9(10):2395-410. doi: 10.1038/nprot.2014.157.
Verdaguer B et al. (1996). Plant Mol Biol. 6:1129-39.
Wang et al. 2014. Nat Biotechnol 32, 947-951. doi.org/10.1038/nbt.2969
Wang et al. 2018. G3 Genes|Genomes|Genetics, Volume 8, Issue 11, 3607-3616, doi.org/10.1534/g3.118.200662
Wright et al. (2001). Plant Cell Rep 20, 429-436. doi.org/10.1007/s002990100318
Youssef et al. 2017. Plant Mol Biol Rep 36, 71-81. doi.org/10.1007/s11105-017-1062-y
Zhang et al, 2016. Nat Commun 7, 12617. doi.org/10.1038/ncomms12617
Zhang et al. 2021. Plant Biotechnol J 19, 1684-1686. doi.org/10.1111/pbi.13647
Zong et al. 2017. Nat Biotechnol 35, 438-440. doi.org/10.1038/nbt.3811
Zong et al. 2018. Nat Biotechnol 36, 950-953. doi.org/10.1038/nbt.4261

## Claims

1. Method for modifying the genome of a plant cell, comprising
a) providing an endonuclease directed to a target site of the gene of interest to the plant cell
b) Providing a nucleic acid molecule comprising a multi-stop cassette, wherein the multi-stop cassette comprises stop codons in all six reading frames to the plant cell,
wherein the endonuclease cuts the genome at the target site, and wherein the multi-stop cassette comprising stop codons in all six reading frames is introduced within the plant cell's genome during repair of the genome at the cut induced by the endonuclease.

2. The method of claim 1, wherein the plant is a cereal.

3. The method of any one of claims 1 or 2, wherein the nucleic acid molecule is provided in a linear form.

4. The method of any one of claims 1 to 3, wherein the length of the multi-stop cassette is at most 20 more preferably at most 12 nucleotides.

5. The method of any one of claims 1 to 4, wherein the sequence of the multi-stop cassette is selected from the sequences of Table 1 (SEQ ID NO: 1 to SEQ ID NO: 64), in particular SEQ ID NO: 1 or SEQ ID NO: 2.

6. The method of any one of claims 1 to 5, wherein the nucleic acid molecule is a double stranded DNA molecule, or a single stranded DNA molecule.

7. The method of any one of claims 1 to 6, further comprising a step of screening the plant cells for identifying cells in the genome of which the multi-stop cassette comprising stop codons in all six frames has been introduced at the target site.

8. The method of any one of claims 1 to 7, wherein the plant is bread wheat.

9. The method of claim 8, wherein the endonuclease targets and cuts a site of interest on all three genomes of the wheat, therefore resulting in integration of the multi-stop cassette in all three genomes, or wherein the endonuclease targets and cuts a site of interest that is present on two genomes of wheat, and not on the third one, therefore resulting in integration of the multi-stop cassette in the two genomes only or wherein the endonuclease targets and cuts a site of interest that is present on one genome of wheat only, and not on the other two, therefore resulting in integration of the multi-stop cassette in one genome only.

10. A method for generating a plant, comprising performing the method of any one of claims 1 to 9 and regenerating a plant from the cells to which the endonuclease and the nucleic acid molecule comprising the multi-stop cassette have been provided.

11. The method of claim 10, wherein the plant is regenerated from isolated cells in the genome of which the multi-stop cassette has been integrated.

12. A plant containing a multi-stop cassette in its genome, wherein the multi-stop cassette comprises stop codons is all six frames of readings and wherein the length of the multi-stop cassette is 20 bases at most, wherein the plant is homozygous for the multi-stop cassette.

13. The plant of claim 12, which is wheat, wherein the multi-stop cassette is integrated in all three genomes of the cereal.

14. A nucleic acid comprising or consisting in a sequence selected from the sequences depicted in Table 1.

15. A method for evaluating the role of a gene of interest in a cereal, comprising
(a) Performing the method of any one of claims 1 to 11, to introduce a multi-stop cassette within the gene of interest
(b) Regenerating a plant comprising the multi-stop cassette introduced within the gene of interest,
(c) Optionally obtaining a plant homozygous for the multi-stop cassette introduced within the gene of interest
(d) Comparing the phenotype of the plant of (b) or (c) with the phenotype of an isogenic plant not having the multi-stop cassette introduced within the gene of interest,
thereby determining the role of the gene of interest as inducing the difference of the phenotypes compared in (d).
